# EUROPÄISCHE PATENTSCHRIFT

(11) **EP 4 039 213 B1**
(45) Veröffentlichungstag und Bekanntmachung des Hinweises auf die Patenterteilung: **03.04.2024**
(21) Anmeldenummer: 22154741.7
(22) Anmeldetag: 02.02.2022
(51) Int. Cl.: A61B 18/14, A61B 18/00

(54) **ELEKTRODENINSTRUMENT FÜR EIN RESEKTOSKOP UND RESEKTOSKOP**
RESECTOSCOPE AND ELECTRODE INSTRUMENT FOR A RESECTOSCOPE
INSTRUMENT À ÉLECTRODES POUR UN RÉSECTOSCOPE ET RÉSECTOSCOPE

(30) Priorität: 05.02.2021 DE 102021102736
(43) Veröffentlichungstag der Anmeldung: 10.08.2022
(73) Patentinhaber: Olympus Winter & Ibe GmbH, 22045 Hamburg (DE)
(72) Erfinder: Brockmann, Christian, 21279 Hollenstedt (DE); KNOPF, Christoph, 23554 Lübeck (DE); Offt, Andreas, 21465 Reinbek (DE)
(74) Vertreter: Hoener, Matthias

(56) Entgegenhaltungen:
- DE-A1- 10 248 839
- DE-A1- 10 258 730
- DE-A1-102013 109 505
- DE-A1-102018 127 919

## Beschreibung

Die Erfindung betrifft ein Elektrodeninstrument für ein Resektoskop gemäß dem Oberbegriff des Anspruchs 1. Des Weiteren betrifft die Erfindung ein Resektoskop gemäß dem Anspruch 12.

Hochfrequenzinstrumente wie das hier beschriebene Resektoskop werden in der Medizin für die Behandlung von Körpergewebe und im Besonderen zum Entfernen oder Manipulieren dieses Gewebes verwendet. Dabei stellen typische Anwendungen solche in der Urologie dar. Als ein Beispiel hierfür ist die Prostataresektion zu nennen. Bei einem zum Einsatz kommenden Hochfrequenzwerkzeug kann es sich um eine Elektrode bzw. eine HF-Elektrode handeln, die an einem Hochfrequenzgenerator angeschlossen wird, wobei der Generator mit einem Schalter von einem Operateur aktiviert und deaktiviert werden kann. Der hochfrequente Strom führt dazu, dass sich an der Elektrode ein Plasma bildet. Aufgrund der eingebrachten thermischen Energie und der Wechselwirkung des Plasmas mit dem Gewebe eignen sich diese HF-Elektroden besonders gut für die Behandlung von Gewebe.

Zum Manipulieren des Gewebes kann die Elektrode als Schneidschlinge oder als Knopfelektrode, aber auch als Nadel, Roller, Band, etc., ausgebildet sein, die sich bei angeschalteter Hochfrequenzspannung sehr leicht und beinahe ohne Widerstand durch das zu entfernende Körpergewebe führen lässt. Neben dem Schneiden von Körpergewebe kann das Gewebe auch noch andersartig manipuliert werden. Dazu ist es vorgesehen, dass verschiedene Hochfrequenzwerkzeuge bzw. verschiedene Elektroden für die Behandlung eingesetzt werden.

Die Elektrode ist über ein Elektrodeninstrument mit einem Transporteur bzw. einem Arbeitselement des Resektoskopes lösbar verrastet. Während der Behandlung des Körpergewebes wird das Elektrodeninstrument mit der Elektrode entlang einer Längsrichtung des Resektoskopes bewegt. Je nachdem, ob es sich bei dem Resektoskop um ein aktives oder passives Resektoskop handelt, ist der Transporteur bzw. der Schlitten mit einer Druckfeder bzw. einer Zugfeder mit einem eine Griffeinheit aufweisenden Hauptkörper verbunden.

Bekannte Elektrodeninstrumente weisen eine gegabelte Grundform auf. Dabei tragen zwei Gabelrohre am distalen Ende jeweils ein Ende der Elektrode. Diese Gabelrohre werden in Richtung des proximalen Endes zusammengeführt und münden in ein gemeinsames Elektrodenhüllrohr. Bei dieser Ausführungsform eines Elektrodeninstrumentes wird der aktive Strompfad bzw. der Aktiv-Kontakt der Elektrode durch das Innere des Elektrodenhüllrohres geleitet, während die Rückführung des Strompfads bzw. der Return-Kontakte über die Außenhülle des Hüllrohres geleitet wird. Die Aufgabelung des Elektrodeninstrumentes erfolgt für gewöhnlich im distalen Endbereich des Resektoskopes. Ein Elektrodeninstrument, wobei von der Elektrode zwei Elektrodenhüllrohre zum proximalen Ende führen, wird in DE 10 2013 109505 A1 offenbart.

Neben dem Elektrodeninstrument ist es außerdem denkbar, dass weitere Komponenten, wie beispielsweise eine Optik, durch den Transporteur geführt werden. Diese Optik kann als Stablinsensystem oder als Lichtleiter ausgebildet sein, die es dem Operateur ermöglichen, während der Operation mittels einer Kamera oder direkt durch ein Okular einen Blick auf den Behandlungsraum im Inneren des Körpers zu werfen. Insbesondere bei dem Einsatz der Elektrode ist es von herausragender Bedeutung, dass dem Operateur ein uneingeschränkter Blick auf den Schneidvorgang zur Verfügung steht. Dementsprechend ist ein distales Ende der Optik derart ausgerichtet, dass das Blickfeld des Operateurs direkt auf die Elektrode gerichtet ist und die Bewegung der Elektrode an die Bewegung der Optik gekoppelt ist.

Bei dem Schneiden des Gewebes kann sowohl entstehendes Gasblasen als auch Blutungen die Sicht des Operateurs eintrüben. Um dem entgegenzuwirken, wird der Bereich vor dem distalen Ende des Resektoskop durch eine Spülflüssigkeit freigespült. Die Flüssigkeit wird dazu durch den Schaft des Resektoskopes geleitet und tritt am distalen Ende aus. Dabei ist es für eine klare Sicht wesentlich, dass sich die austretende Spülflüssigkeit möglichst laminar verhält. Eine optimierte Strömung ist insbesondere erreichbar, wenn am Austrittsende, d. h. am distalen Ende des Resektoskopes, die Strömung der Spülflüssigkeit nicht gestört wird.

Eine derartige Störung kann allerdings durch die Aufgabelung des Elektrodeninstrumentes erfolgen. Die durch den Schaft strömende Flüssigkeit trifft auf die Aufgabelung und verwirbelt. Diese Verwirbelungen der Spülflüssigkeit führt zu einer turbulenten Strömung, welche besonders nachteilig für die Sicht des Operateurs ist. Ein Versetzen der Aufgabelung entlang des Elektrodeninstrumentes hätte zur Folge, dass dieses nicht mehr die nötige mechanische Stabilität aufweisen würde. Insgesamt ist die gegabelte Grundform notwendig, um die Elektrode mit den stromführenden Kontakten bzw. dem Transporteur zu verbinden.

Der Erfindung liegt die Aufgabe zugrunde, ein Elektrodeninstrument sowie ein Resektoskop zu schaffen, durch welches die oben genannten Probleme gelöst werden.

Eine Lösung dieser Aufgabe wird durch die Merkmale des Anspruchs 1 beschrieben.

Demnach ist es vorgesehen, dass von einer Elektrode zwei Elektrodenhüllrohre zum proximalen Endbereich des Elektrodeninstrumentes führen, die jeweils einen elektrischen Kontakt, nämlich einen Aktiv-Kontakt sowie einen Return-Kontakt, aufweisen. Durch diese zwei durchgehenden Elektrodenhüllrohre kann auf eine Aufgabelung der Elektrodenhüllrohre verzichtet werden. Eine Verwirbelungen einer Spülflüssigkeit, die von einem proximalen Ende zu einem distalen Ende des Elektrodeninstrumentes entlang der Elektrodenhüllrohre strömt, wird dadurch vermieden.

Weiter wird es vorgesehen, dass der Aktiv-Kontakt bzw. der entsprechende elektrische Leiter elektrisch isoliert innerhalb eines Elektrodenhüllrohres angeordnet ist und der Return-Kontakt bzw. der entsprechende elektrische Leiter durch die äußere Hülle der Elektrodenhüllrohre gebildet ist bzw. mit der Hülle mindestens eines Elektrodenhüllrohres leitfähig verbunden ist. Durch diese Aufteilung der beiden Kontakte lässt sich die Stromführung in dem Elektrodeninstrument besonders einfach, effizient sowie sicher gestalten. Auch die elektrische Kontaktierung innerhalb des Transporteurs wird durch diese Stromführung erleichtert.

Erfindungsgemäß wird das Elektrodeninstrument so gestaltet, dass der Aktiv-Kontakt zur elektrischen Kontaktierung der Elektrode am proximalen Ende des Elektrodeninstrumentes aus einem der beiden Elektrodenhüllrohre herausragt, bzw. dass mindestens eine Kontaktfläche des Aktiv-Kontaktes außerhalb des Elektrodenhüllrohres frei zugänglich ist. Der elektrische Leiter, der den Aktiv-Kontakt bildet, kann vorzugsweise auch durch das entsprechende andere Elektrodenhüllrohr geführt werden, wobei dort dieser Leiter wenigstens einige Zentimeter vor dem proximalen Ende Elektrodenhüllrohres innerhalb desselben endet.

Darüber hinaus ist es erfindungsgemäß vorgesehen, dass wenigstens distale und proximale Abschnitte der Elektrodenhüllrohre parallel zueinander verlaufen, wobei vorzugsweise die Elektrodenhüllrohre über ihre gesamte Länge parallel zueinander verlaufen. Demnach sind die Elektrodenhüllrohre auch parallel bezüglich des Resektoskopes bzw. des Schafts und der Optik ausgerichtet. Durch diese parallele Ausrichtung der Elektrodenhüllrohre und insbesondere aller Komponenten ergibt sich ein optimiertes Strömungsbild der Spülflüssigkeit. Diese Parallelität der Elektrodenhüllrohre führt somit zu einer besonders klaren Sicht auf den Ort der Behandlung.

Es ist erfindungsgemäß auch denkbar, dass Längsachsen proximaler und distaler Abschnitte eines der Elektrodenhüllrohre in einer gemeinsamen ersten Ebene liegen und Längsachsen proximaler und distaler Abschnitte des anderen Elektrodenhüllrohres in einer gemeinsamen zweiten Ebene liegen. Die erste und die zweite Ebene sind dabei parallel zueinander ausgerichtet und weisen somit über die gesamte Länge der Elektrodenhüllrohre einen gleichen Abstand zueinander auf. Die proximalen Abschnitte sind somit gegenüber den distalen Abschnitten nicht versetzt, was zu einer Störung des Spülflüssigkeitsstroms führen würde. Vielmehr wird durch diese Parallelität der Abschnitte eine geradlinige Führung der genannten Abschnitte der Elektrodenhüllrohre über die gesamte Länge des Elektroinstrumentes erreicht. Durch diese Geometrie können optimale Bedingungen für eine wenigstens nahezu störungsfreie Strömung geschaffen werden.

Ein weiteres bevorzugtes Ausführungsbeispiel der Erfindung sieht es vor, dass die proximalen Abschnitte der Elektrodenhüllrohre, insbesondere der Aktiv-Kontakt und der Return-Kontakt, über eine Länge von mindestens 24 mm, vorzugsweise mindestens 40 mm, gerade ausgebildet sind. Durch diese geradlinige Ausbildung der Endabschnitte gestaltet sich die Kontaktierung zwischen dem Elektrodeninstrument und dem Transporteur als besonders vorteilhaft bzw. einfach. Durch diese Dimensionierung lässt sich dieses Elektrodeninstrument besonders einfach mit dem Transporteur koppeln bzw. entkoppeln. Ein weiterer Vorteil gegenüber bestehenden Systemen besteht darin, dass durch den langen geraden Abschnitt auf einen flexiblen Bereich verzichtet werden kann. Hierdurch wird die Betätigungskraft verringert, da weniger Kraft für elastische Verformung aufgewandt werden muss. Zusätzlich wirken keine Querkräfte auf Dichtungen. Außerdem ermöglicht es einen größeren axialen Bereich, auf dem die Elektrode im Schlitten kontaktiert werden kann.

Besonders bevorzugt kann es vorgesehen sein, dass die distalen Abschnitte der Elektrodenhüllrohre über eine Länge von mindestens 100 mm, vorzugsweise mindestens 200 mm, geradlinig ausgebildet sind. Diese geradlinige Ausgestaltung der distalen Abschnitte führt optimaler Weise zu einer laminaren Strömung der Spülflüssigkeit innerhalb des Schaftes. Durch diese geradlinige sowie parallele Führung der Elektrodenhüllrohre wenigstens über die gesamte Länge des distalen Endabschnitts wird das Strömungsverhalten der Spülflüssigkeit so gut wie nicht gestört, wodurch Verwirbelungen vor der Optik unterbunden werden.

Vorzugsweise sieht es die Erfindung weiter vor, dass distale Längsachsen distaler Abschnitte der Elektrodenhüllrohre gegenüber proximalen Längsachsen proximaler Abschnitte der Elektrodenhüllrohre jeweils mindestens einen Versatz aufweisen. Dieser Versatz der Längsachsen entlang der Längsachse des Resektoskopes kann beispielsweise S-förmig auf einer Länge von maximal 30 mm, bevorzugt 15 mm, ausgebildet sein. Durch diesen Versatz der proximalen sowie distalen Abschnitte wird die Elektrode besonders vorteilhaft getragen bzw. positioniert und gleichzeitig werden die elektrischen Kontakte am proximalen Ende des Elektrodeninstrumentes in eine vorteilhafte Position gebracht, um sie mit dem Transporteur zu koppeln. Dabei sind die Versätze tendenziell eher in Richtung des proximalen Endes des Elektrodeninstrumentes angeordnet, um den Einfluss auf das Strömungsprofil am distalen Ende zu minimieren.

Eine besonders vorteilhafte Ausgestaltung der Erfindung sieht es vor, dass der Versatz 2,5 mm bis 3,5 mm, vorzugsweise 2,7 mm bis 3,1 mm, insbesondere 2,9 mm, beträgt. Es hat sich gezeigt, dass ein derartiger Versatz für die elektrische Kontaktierung, die mechanische Stabilität, aber auch für eine laminare Strömung der Spülflüssigkeit besonders vorteilhaft ist.

Insbesondere kann es die Erfindung vorsehen, dass der Abstand der Elektrodenhüllrohre im proximalen Bereich 5,4 mm bis 5,8 mm, vorzugsweise 5,6 mm, und/oder der Abstand der Elektrodenhüllrohre im distalen Bereich 5,4 mm bis 5,8 mm, vorzugsweise 5,6 mm, beträgt. Durch diese Beabstandung der Elektrodenhüllrohre ergibt sich zum einen eine besonders effiziente Ausnutzung des Raumes des Schaftes des Resektoskopes und zum anderen bleibt der Spülflüssigkeit sowie weiteren Komponenten, wie beispielsweise der Optik, ausreichend Raum im Inneren des Schaftes. Des Weiteren besteht ein Vorteil der Beabstandung im distalen Abschnitt darin, dass eine Positionierung der Elektrodenhüllrohre zwischen Innen- und Außenschaft möglich ist. Eine Beabstandung im proximalen Abschnitt weist den Vorteil auf, dass zusätzlich zum Versatz der Abstand zur Optik vergrößert werden kann, um Platz für die Kontaktierung im Schlitten zu schaffen. Ein gleicher Abstand der distalen und proximalen Abschnitte weist auch den Vorteil auf, dass sich die Herstellung des Elektroneninstrumentes vereinfacht, da die Elektrodenhüllrohre nur in eine Raumrichtung verschwenkt werden müssen.

Ein weiteres wesentliches Merkmal der Erfindung kann darin bestehen, dass der Durchmesser der Elektrodenhüllrohre 1 mm bis 1,4 mm, vorzugsweise 1,2 mm, beträgt, wobei ein Querschnitt der Elektrodenhüllrohre wenigstens abschnittsweise, vorzugsweise über die gesamte Länge der Elektrodenhüllrohre, kreisförmig bzw. kreisartig ausgebildet ist. Diese Dimensionierung der Hüllrohre ist besonders vorteilhaft, da sie über die gesamte Länge des Instrumentes eine ausreichende mechanische Stabilität bietet. Des Weiteren reicht diese Dimensionierung aus, um die Schneidelektrode mit der entsprechenden elektrischen Energie zu versorgen. Des Weiteren ist es möglich mit dieser Dimensionierung die Elektrodenhüllrohre zwischen Innenschaft und Außenschaft zu positionieren und zwar bei optimaler Abwägung von Flowquerschnitten und Fertigbarkeit der mechanischen Komponenten des Resektoskopes. Außerdem lässt sich das Elektrodeninstrument mit einer derartigen Dimensionierung durch den Operateur besonders gut handhaben, insbesondere mit dem Transporteur koppeln bzw. entkoppeln. Als besonders Vorteilhaft gestaltet es sich, dass das Abdichten auf dem größten Durchmesser stattfindet. Dabei muss kein größeres Element durch die Dichtung im Transporteur geführt werden. Insbesondere kann die Vercrimpung der Elektrodenhüllrohre derart erfolgen, dass von der kreisartigen Querschnittsform nur geringfügig abgewichen wird, um das Strömungsverhalten der Spülflüssigkeit nicht zu beeinflussen.

Weiter ist es denkbar, dass alle Komponenten der Elektrode, insbesondere des Return-Kontaktes, im proximalen Bereich sich innerhalb einer verlängerten umhüllenden Zylinderfläche der Hüllrohre befinden und diese radial nicht überragen.

Darüber hinaus kann es eine vorteilhafte Weiterbildung der vorliegenden Erfindung darstellen, dass die Elektrodenhüllrohre über mindestens ein Führungselement bzw. Führungsblech, insbesondere zwei Führungselemente im distalen Abschnitt, vorzugsweise mit einem ersten Führungselement im distalen und einem zweiten Führungselement im proximalen Abschnitt, verbunden sind. Ein derartiges stegartiges Führungselement verbindet die beiden Elektrodenhüllrohre, sodass deren relative Lage wenigstens weitestgehend auch bei mechanischer Krafteinwirkung konstant bleibt. Darüber hinaus kann es vorgesehen sein, dass ein Querschnitt des mindestens einen Führungselementes gekrümmt ist bzw. ein Kreissegment bildet, sodass sich das Führungselement der Innenwandung des Außenschaftes, Außenwandung der Optik oder Innen- / Außenwandung des Innenschafts bzw. der Optik anpasst. Das Führungselement des Elektrodeninstrumentes fixiert dieses derartig, dass das Elektrodeninstrument nur noch in axialer Richtung des Schaftes / der Optik bewegbar ist.

Das Führungselement ist derart ausgebildet, dass die Strömung der Spülflüssigkeit möglichste wenig gestört wird. Dazu ist das Führungselement aus dem Inflow in den Outflow versetzt. Die Enden des mindestens einen Führungselementes sind fest mit der äußeren Hülle der Elektrodenhüllrohre verbunden. Dabei können die Enden der Führungselemente mit den Hüllrohren fest verbunden sein, nämlich entweder verklebt, verschweißt, vercrimpt, verlötet, verrastet oder dergleichen sein. Vorzugsweise sind die Führungselemente metallisch ausgebildet. Gleichermaßen können sie aber auch aus einem Kunststoff oder einem Verbundwerkstoff hergestellt sein.

Eine weitere Lösung der eingangs genannten Aufgabe wird durch die Merkmale des Anspruchs 14 beschrieben. Demnach ist es vorgesehen, dass ein Resektoskop ein Elektrodeninstrument gemäß den Ansprüchen 1-13 aufweist. Durch derartige Resektoskope ist der Bereich der Behandlung sowohl zuverlässig mit einem HF-Plasma versorgbar, als auch durch eine möglichst laminare Strömung einer Spülflüssigkeit gut einsehbar.

Ein bevorzugtes Ausführungsbeispiel für ein Elektrodeninstrument wird im Folgenden anhand der Zeichnung näher beschrieben. In dieser zeigen:
- Fig. 1: eine schematische Darstellung eines Resektoskopes,
- Fig. 2: eine schematische, perspektivische Darstellung eines Elektrodeninstrumentes,
- Fig. 3: eine Sicht auf das Elektrodeninstrument gemäß Fig. 2,
- Fig. 4: eine Seitenansicht des Elektrodeninstrumentes gemäß Fig. 2, und
- Fig. 5: eine Sicht auf ein distales Ende des Elektrodeninstrumentes gemäß Fig. 2.

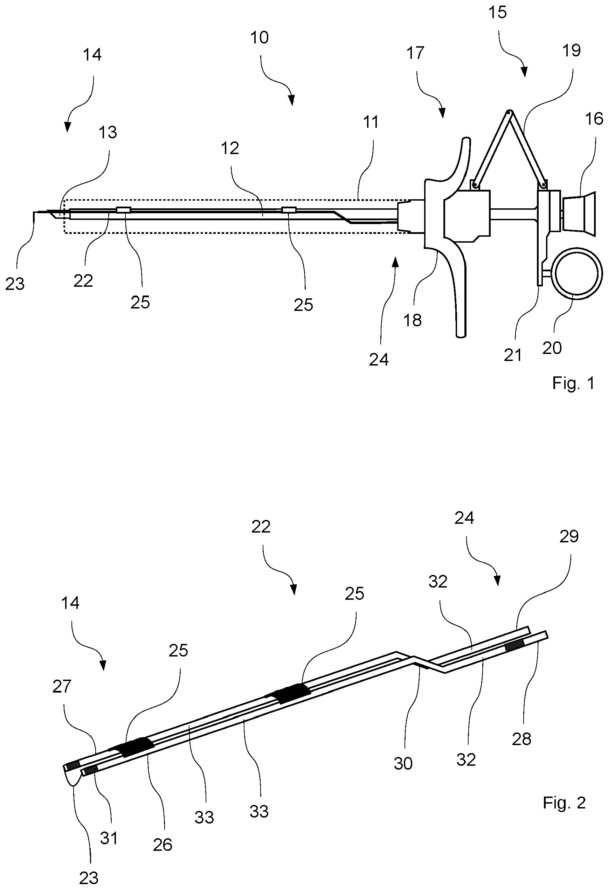

In der Fig. 1 ist ein mögliches Ausführungsbeispiel eines Resektoskopes 10 dargestellt. Bei diesem Resektoskop 10 ist ein äußerer Schaft 11, hier nur durch Strichlinien angedeutet, über einen inneren Schaft 12 geschoben. Der innere Schaft 12 dient der Aufnahme bzw. der Führung einer Optik 13, die sich von einem distalen Ende 14 zu einem proximalen Ende 15 des Resektoskopes 10 erstreckt. Am proximalen Ende 15 steht einem Benutzer ein Okular 16 zur Verfügung, um durch die Optik 13 den zu operierenden Bereich vor dem distalen Ende 14 zu beobachten.

Ein wesentlicher Bestandteil des Resektoskopes 10 stellt der Transporteur 17 oder auch Arbeitselement dar. Dieser Transporteur 17 weist unter anderem ein erstes Griffmittel 18 auf und ist über ein Federelement 19 mit einem zweiten Griffmittel 20 sowie einer Optikplatte 21 verbunden.

Außerdem erstreckt sich entlang des inneren Schaftes 12 ein Elektrodeninstrument 22 von dem distalen Ende 14 des Resektoskopes 10 bis zu dem Transporteur 17. Am distalen Ende 14 weist das Elektrodeninstrument 22 eine Elektrode 23 auf. Diese Elektrode 23 ist über einen nicht dargestellten HF-Generator mit elektrischer Energie beaufschlagbar, die dazu dient Gewebe zu manipulieren.

Das Elektrodeninstrument 22 ist mit einem proximalen Ende 24 in dem Transporteur 17 verrastet. Dadurch lässt sich zum einen das Elektrodeninstrument 22 auf eine einfache Art und Weise von dem Transporteur 17 entkoppeln bzw. an dem Transporteur 17 koppeln und zum anderen mitsamt dem Transporteur 17 entlang der Längsachse des Resektoskopes 10 in distale oder proximale Richtung bewegen.

Das in der Fig. 2 dargestellte Elektrodeninstrument 22 weist erfindungsgemäß zwei Elektrodenhüllrohre 26, 27 auf. Diese Elektrodenhüllrohre 26, 27 sind im Wesentlichen geradlinig ausgebildet. Die Elektrodenhüllrohre 26, 27 verlaufen über ihre gesamte Länge, d. h. vom distalen Ende 14 bis zum proximalen Ende 24, parallel zueinander. Dabei beträgt der Abstand zwischen den Elektrodenhüllrohren 26, 27 zwischen 5,4 mm bis 5,8 mm, vorzugsweise 5,6 m. An den distalen Enden sind die beiden Elektrodenhüllrohre 26, 27 durch die Elektrode 23 mechanisch miteinander verbunden sowie elektrisch durch den Isolator 31 voneinander isoliert. Durch Beaufschlagung des Elektrodeninstrumentes 22 mit einer HF-Spannung bildet sich um die hier als Schneidschlinge ausgebildete Elektrode 23 ein Plasma. Die elektrische Kontaktierung des Elektrodeninstrumentes 22 erfolgt über die beiden proximalen Enden der Elektrodenhüllrohre 26, 27. Während ein Aktiv-Kontakt 28 isoliert im Inneren des Elektrodenhüllrohres 26 von dem proximalen Ende 24 zur Elektrode 23 verläuft, bildet eine äußere Hülle des Elektrodenhüllrohres 27 einen Return-Kontakt 29. Diese beiden Kontakte 28, 29 werden wie bereits beschrieben in den Transporteur 17 gesteckt und über entsprechende Leitungen mit dem hier nicht dargestellten HF-Generator elektrische verbunden.

Die Elektrodenhüllrohre 26, 27 weichen von ihrer geradlinigen Form dahingehend ab, dass proximale Abschnitte 32 gegenüber distale Abschnitte 33 jeweils einen Versatz 30 aufweisen. Durch diesen Versatz 30 sind Längsachsen der proximalen Abschnitte 32 und der distalen Abschnitte 33 der einzelnen Elektrodenhüllrohre 26, 27 gegeneinander parallel verschoben, wobei der proximale Abschnitt 32 und der distale Abschnitt 33 des Elektrodenhüllrohres 26 in einer ersten Ebene liegen, welche parallel ausgerichtet ist gegenüber einer zweiten Ebene, die durch den proximalen Abschnitt 32 und den distalen Abschnitt 33 des Elektrodenhüllrohres 27 gebildet ist. Dabei kann der Versatz 30 zwischen den verschobenen Längsachsen bzw. der Versatz 30 2,5 mm bis 3,5 mm, andere 2,7 mm bis 3,1 mm, vorzugsweise 2,9 mm, betragen. Dieser Versatz 30 kann sowohl stufenartig aber auch S-förmig ausgebildet sein.

Bei dem in den Fig. 2-4 dargestellten Ausführungsbeispiel des Elektrodeninstrumentes 22 ist es vorgesehen, dass der distale geradlinige Abschnitt 33 der Elektrodenhüllrohre 26, 27 mindestens eine Länge von 100 mm, vorzugsweise von 200 mm, aufweist. Durch diese Ausgestaltung wenigstens des distalen Abschnitts 33 der Elektrodenhüllrohre 26, 27 bildet sich eine besonders vorteilhafte, laminare Strömung der Spülflüssigkeit in dem Schaft 11 aus. Der geradlinige proximale Abschnitt 32 der Elektrodenhüllrohre 26, 27 weist bevorzugt eine Länge von 24 mm, vorzugsweise 40 mm, auf. Durch diese Dimensionierung lassen sich die Kontakte 28, 29 besonders einfach und sicher handhaben bzw. mit dem Transporteur 17 koppeln.

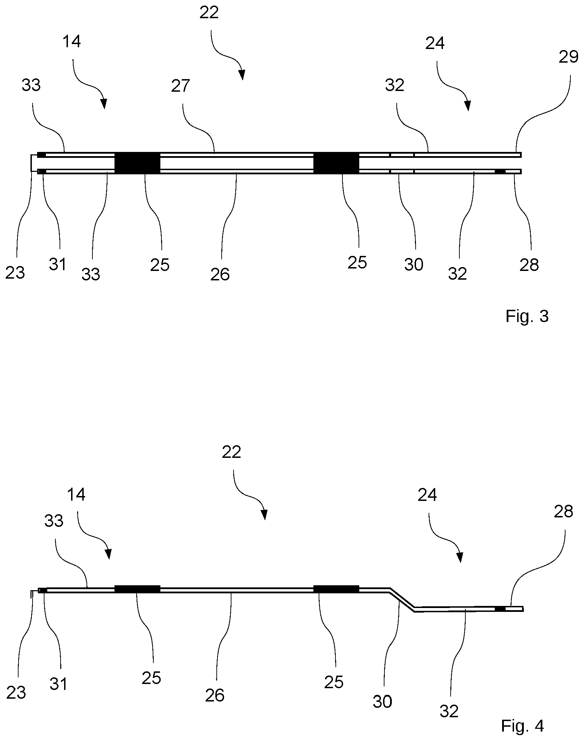

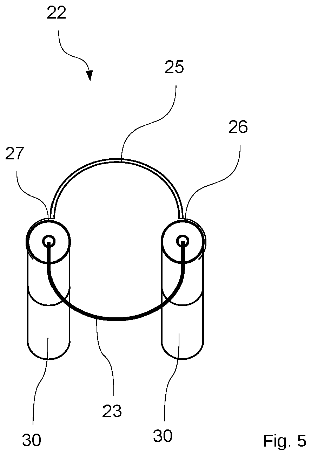

Für eine zusätzliche mechanische Stabilität können die beiden Elektrodenhüllrohre 26, 27, wie in Fig. 2 und 3 beispielhaft dargestellt, über Führungselemente 25 miteinander gekoppelt sein. Diese Führungselemente 25 dienen sowohl der mechanischen Stabilisierung der Elektrodenhüllrohre 26, 27 untereinander, aber vor allen Dingen auch der Führung des Elektrodeninstrumentes 22 auf dem inneren Schaft 12. Bei der Verwendung des Resektoskopes 10 liegen die Führungselemente 25 auf einer äußeren Mantelfläche des inneren Schaftes 12 und gleiten bei entsprechender Bewegung entlang der Schaftachse. Die Fig. 5 lässt erkennen, dass der Querschnitt der Führungselemente 25 kreissegmentartig ausgebildet ist und sich dadurch besonders vorteilhaft der äußeren Form des inneren Schaftes 12 anschließen kann. Außerdem ist aus Fig. 5 erkennbar, dass die Führungselemente 25 mit ihren Randbereichen an den Elektrodenhüllrohren 26, 27 befestigt sind bzw. diese Randbereiche wenigstens teilweise um die Elektrodenhüllrohre 26, 27 herumreichen. Diese Führungselemente 25 können entweder aus einem metallischen Material oder alternativ aus einem Kunststoff oder Verbundwerkstoff hergestellt werden. Aufgrund der Positionierung der Führungselemente 25 oberhalb des inneren Schaftes 12 bleibt die durch den Schaft 11 strömende Spülflüssigkeit unbeeinflusst von diesen Blechen 25.

Um den begrenzten Innenraum des Schaftes 11 optimal auszunutzen, beträgt der Durchmesser der Elektrodenhüllrohr 26, 27 1,0 mm bis 1,4 mm, vorzugsweise 1,2 mm.

Neben dem hier beispielhaft dargestellten Resektoskop 10 ist es auch denkbar, dass das erfindungsgemäße Elektrodeninstrument 22 mit einem andersartig ausgebildeten Resektoskop verbunden wird.

### Bezugszeichenliste:

| | | | |
|---|---|---|---|
| 10 | Resektoskop | 29 | Return-Kontakt |
| 11 | Äußerer Schaft | 30 | Versatz |
| 12 | Innerer Schaft | 31 | Isolator |
| 13 | Optik | 32 | Proximaler Abschnitt |
| 14 | Distales Ende des Resektoskopes | 33 | Distaler Abschnitt |
| 15 | Proximales Ende des Resektoskopes | | |
| 16 | Okular | | |
| 17 | Transporteur | | |
| 18 | Griffmittel | | |
| 19 | Federelement | | |
| 20 | Griffmittel | | |
| 21 | Optikplatte | | |
| 22 | Elektrodeninstrument | | |
| 23 | Elektrode | | |
| 24 | Proximales Ende des Elektrodeninstrumentes | | |
| 25 | Führungselement | | |
| 26 | Elektrodenhüllrohr | | |
| 27 | Elektrodenhüllrohr | | |
| 28 | Aktiv- Kontakt | | |

## Patentansprüche

1. Elektrodeninstrument (22) für ein Resektoskop (10), wobei das Elektrodeninstrument (22) an einem distalen Ende (14) eine Elektrode (23) aufweist und an einem proximalen Ende (24) über einen elektrischen Kontakt lösbar mit einem Transporteur (17) des Resektoskopes (10) koppelbar ist, wobei von der Elektrode (23) zwei Elektrodenhüllrohre (26, 27) zum proximalen Ende (24) führen mit jeweils einem elektrischen Kontakt, nämlich einem Aktiv-Kontakt (28) und einem Return-Kontakt (29), **dadurch gekennzeichnet, dass** der elektrische Aktiv-Kontakt (28) elektrisch isoliert innerhalb eines Elektrodenhüllrohrs (26) angeordnet ist und der elektrische Return-Kontakt (29) durch eine Hülle eines Elektrodenhüllrohrs (27) gebildet ist bzw. mit der Hülle mindestens eines Elektrodenhüllrohrs leitfähig verbunden ist und der Aktiv-Kontakt (28) zur elektrischen Kontaktierung am proximalen Ende (24) aus dem Elektrodenhüllrohr (26) herausragt.

2. Elektrodeninstrument (22) nach Anspruch 1, wobei wenigstens distale und proximale Abschnitte (32, 33) der Elektrodenhüllrohre (26, 27) parallel zueinander verlaufen, vorzugsweise dass die Elektrodenhüllrohre (26, 27) über ihre gesamte Länge parallel zueinander verlaufen.

3. Elektrodeninstrument (22) nach Anspruch 1 oder 2, wobei Längsachsen proximaler und distaler Abschnitte (32, 33) des Elektrodenhüllrohres (26) in einer gemeinsamen ersten Ebene liegen und Längsachsen proximaler und distaler Abschnitte (32, 33) des Elektrodenhüllrohres (27) in einer gemeinsamen zweiten Ebene liegen, wobei die erste und die zweite Ebene parallel zueinander ausgerichtet sind und somit der Abstand der Elektrodenhüllrohre (26, 27) über deren gesamte Länge gleich ist.

4. Elektrodeninstrument (22) nach einem der vorangegangenen Ansprüche, wobei die proximalen Abschnitte (32) der Elektrodenhüllrohre (26, 27), insbesondere der Aktiv-Kontakt (28) und der Return-Kontakt (29), über eine Länge von mindestens 24 mm, vorzugsweise mindestens 40 mm, gerade ausgebildet sind und/oder die distalen Abschnitte (33) der Elektrodenhüllrohre (26, 27) über eine Länge von mindestens 100 mm, vorzugsweise mindestens 200 mm, gerade ausgebildet sind.

5. Elektrodeninstrument (22) nach einem der vorangegangenen Ansprüche, wobei distale Längsachsen distaler Abschnitte (33) der Elektrodenhüllrohre (26, 27) gegenüber proximalen Längsachsen proximaler Abschnitte (32) der Elektrodenhüllrohre (26, 27) jeweils mindestens einen Versatz (30) aufweisen.

6. Elektrodeninstrument (22) nach Anspruch 5, Ansprüche, wobei der Versatz (30) 2,5 mm bis 3,5 mm, insbesondere 2,7 mm bis 3,1 mm, vorzugsweise 2,9 mm, beträgt.

7. Elektrodeninstrument (22) nach Anspruch 5 oder 6, wobei der Versatz (30) S-förmig auf einer Länge von maximal 30 mm, bevorzugt maximal 15 mm, ausgebildet ist.

8. Elektrodeninstrument (22) nach einem der vorangegangenen Ansprüche, wobei der Abstand der Elektrodenhüllrohre (26, 27) im proximalen Bereich (33) 5,4 mm bis 5,8 mm, vorzugsweise 5,6 mm, und/oder der Abstand der Elektrodenhüllrohre (26, 27) im distalen Bereich (33) 5,4 mm bis 5,8 mm, vorzugsweise 5,6 mm, beträgt.

9. Elektrodeninstrument (22) nach einem der vorangegangenen Ansprüche, wobei der Durchmesser der Elektrodenhüllrohre (26, 27) 1 mm bis 1,4 mm, vorzugsweise 1,2 mm, beträgt, wobei ein Querschnitt der Elektrodenhüllrohre (26, 27) wenigstens abschnittsweise, vorzugsweise über die gesamte Länge der Elektrodenhüllrohre (26, 27), kreisförmig bzw. kreisartig ausgebildet ist.

10. Elektrodeninstrument (22) nach einem der vorangegangenen Ansprüche, wobei alle Komponenten der Elektrode (23), insbesondere des Return-Kontakts (29) im proximalen Bereich sich innerhalb einer verlängerten umhüllenden Zylinderfläche der Hüllrohre (26, 27) befinden und diese radial nicht überragen.

11. Elektrodeninstrument (22) nach einem der vorangegangenen Ansprüche, wobei die Elektrodenhüllrohre (26, 27) über mindestens ein Führungselement (25), insbesondere zwei Führungselemente (25) im distalen Abschnitt (33), vorzugsweise mit einem ersten Führungselement (25) im distalen (33) und einem zweiten Führungselement (25) im proximalen Abschnitt (32), verbunden sind.

12. Resektoskop (10) mit einem Elektrodeninstrument (22) nach einem der vorangegangenen Ansprüche 1 bis 11.

## Claims

1. Electrode instrument (22) for a resectoscope (10), wherein the electrode instrument (22) has an electrode (23) at a distal end (14) and is able to be releasably coupled at a proximal end (24) to a working element (17) of the resectoscope (10) via an electrical contact, wherein two electrode casing tubes (26, 27) run from the electrode (23) to the proximal end (24) and each have a respective electrical contact, namely an active contact (28) and a return contact (29), **characterized in that** the electrical active contact (28) is arranged electrically insulated inside an electrode casing tube (26), and the electrical return contact (29) is formed by a casing of an electrode casing tube (27) or is conductively connected to the casing of at least one electrode casing tube, and the active contact (28) protrudes from the electrode casing tube (26) for the electrical contacting at the proximal end (24).

2. Electrode instrument (22) according to Claim 1, wherein at least distal and proximal portions (32, 33) of the electrode casing tubes (26, 27) run parallel to each other, preferably wherein the electrode casing tubes (26, 27) run parallel to each other along their entire length.

3. Electrode instrument (22) according to Claim 1 or 2, wherein longitudinal axes of proximal and distal portions (32, 33) of the electrode casing tube (26) lie in a common first plane, and longitudinal axes of proximal and distal portions (32, 33) of the electrode casing tube (27) lie in a common second plane, wherein the first plane and the second plane are oriented parallel to each other and therefore the distance between the electrode casing tubes (26, 27) is identical along their entire length.

4. Electrode instrument (22) according to one of the preceding claims, wherein the proximal portions (32) of the electrode casing tubes (26, 27), in particular the active contact (28) and the return contact (29), are of a rectilinear configuration along a length of at least 24 mm, preferably at least 40 mm, and/or the distal portions (33) of the electrode casing tubes (26, 27) are of a rectilinear configuration along a length of at least 100 mm, preferably at least 200 mm.

5. Electrode instrument (22) according to one of the preceding claims, wherein distal longitudinal axes of distal portions (33) of the electrode casing tubes (26, 27) each have at least one offset (30) in relation to proximal longitudinal axes of proximal portions (32) of the electrode casing tubes (26, 27).

6. Electrode instrument (22) according to Claim 5, wherein the offset (30) measures 2.5 mm to 3.5 mm, in particular 2.7 mm to 3.1 mm, preferably 2.9 mm.

7. Electrode instrument (22) according to Claim 5 or 6, wherein the offset (30) is S-shaped along a length of at most 30 mm, preferably at most 15 mm.

8. Electrode instrument (22) according to one of the preceding claims, wherein the distance between the electrode casing tubes (26, 27) in the proximal region (33) measures 5.4 mm to 5.8 mm, preferably 5.6 mm, and/or the distance between the electrode casing tubes (26, 27) in the distal region (33) measures 5.4 mm to 5.8 mm, preferably 5.6 mm.

9. Electrode instrument (22) according to one of the preceding claims, wherein the diameter of the electrode casing tubes (26, 27) measures 1 mm to 1.4 mm, preferably 1.2 mm, wherein a cross section of the electrode casing tubes (26, 27) is circular or ring-like at least in some regions, preferably along the entire length of the electrode casing tubes (26, 27).

10. Electrode instrument (22) according to one of the preceding claims, wherein all the components of the electrode (23), in particular of the return contact (29), in the proximal region are located within an extended, enveloping cylinder face of the casing tubes (26, 27) and do not protrude radially beyond same.

11. Electrode instrument (22) according to one of the preceding claims, wherein the electrode casing tubes (26, 27) are connected via at least one guide element (25), in particular two guide elements (25), in the distal portion (33), preferably via a first guide element (25) in the distal portion (33) and a second guide element (25) in the proximal portion (32).

12. Resectoscope (10) having an electrode instrument (22) according to one of Claims 1 to 11.

## Revendications

1. Instrument à électrode (22) pour un résectoscope (10), l'instrument à électrode (22) présentant une électrode (23) à une extrémité distale (14) et pouvant être couplé de manière amovible à une extrémité proximale (24) à un transporteur (17) du résectoscope (10) par l'intermédiaire d'un contact électrique, deux tubes d'enveloppe d'électrode (26, 27, 28) conduisant de l'électrode (23) à l'extrémité proximale (24) avec respectivement un contact électrique, à savoir un contact actif (28) et un contact de retour (29), **caractérisé en ce que** le contact électrique actif (28) est agencé de manière isolée électriquement à l'intérieur d'un tube d'enveloppe d'électrode (26) et le contact électrique de retour (29) est formé par une enveloppe d'un tube d'enveloppe d'électrode (27) ou est relié de manière conductrice à l'enveloppe d'au moins un tube d'enveloppe d'électrode et le contact actif (28) sort du tube d'enveloppe d'électrode (26) à l'extrémité proximale (24) pour établir le contact électrique.

2. Instrument à électrode (22) selon la revendication 1, dans lequel au moins des sections distale et proximale (32, 33) des tubes d'enveloppe d'électrode (26, 27) sont parallèles entre elles, de préférence les tubes d'enveloppe d'électrode (26, 27) sont parallèles entre eux sur toute leur longueur.

3. Instrument à électrode (22) selon la revendication 1 ou 2, dans lequel les axes longitudinaux de sections proximale et distale (32, 33) du tube d'enveloppe d'électrode (26) sont situés dans un premier plan commun et les axes longitudinaux de sections proximale et distale (32, 33) du tube d'enveloppe d'électrode (27) sont situés dans un deuxième plan commun, le premier et le deuxième plan étant orientés parallèlement l'un à l'autre et la distance entre les tubes d'enveloppe d'électrode (26, 27) étant ainsi égale sur toute leur longueur.

4. Instrument à électrode (22) selon l'une quelconque des revendications précédentes, dans lequel les sections proximales (32) des tubes d'enveloppe d'électrode (26, 27), notamment le contact actif (28) et le contact de retour (29), sont réalisées sous forme droite sur une longueur d'au moins 24 mm, de préférence d'au moins 40 mm, et/ou les sections distales (33) des tubes d'enveloppe d'électrode (26, 27) sont réalisées sous forme droite sur une longueur d'au moins 100 mm, de préférence d'au moins 200 mm.

5. Instrument à électrode (22) selon l'une quelconque des revendications précédentes, dans laquelle les axes longitudinaux distaux de sections distales (33) des tubes d'enveloppe d'électrode (26, 27) présentent chacun au moins un décalage (30) par rapport aux axes longitudinaux proximaux de sections proximales (32) des tubes d'enveloppe d'électrode (26, 27).

6. Instrument à électrode (22) selon la revendication 5, dans lequel le décalage (30) est de 2,5 mm à 3,5 mm, notamment de 2,7 mm à 3,1 mm, de préférence de 2,9 mm.

7. Instrument à électrode (22) selon la revendication 5 ou 6, dans lequel le décalage (30) est réalisé en forme de S sur une longueur de 30 mm maximum, de préférence de 15 mm maximum.

8. Instrument à électrode (22) selon l'une quelconque des revendications précédentes, dans lequel la distance entre les tubes d'enveloppe d'électrode (26, 27) dans la zone proximale (33) est de 5,4 mm à 5,8 mm, de préférence de 5,6 mm, et/ou la distance entre les tubes d'enveloppe d'électrode (26, 27) dans la zone distale (33) est de 5,4 mm à 5,8 mm, de préférence de 5,6 mm.

9. Instrument à électrode (22) selon l'une quelconque des revendications précédentes, dans lequel le diamètre des tubes d'enveloppe d'électrode (26, 27) est de 1 mm à 1,4 mm, de préférence de 1,2 mm, une section transversale des tubes d'enveloppe d'électrode (26, 27) étant réalisée au moins par sections, de préférence sur toute la longueur des tubes d'enveloppe d'électrode (26, 27), en forme de cercle ou de manière circulaire.

10. Instrument à électrode (22) selon l'une quelconque des revendications précédentes, dans lequel tous les composants de l'électrode (23), notamment du contact de retour (29) dans la zone proximale, se trouvent à l'intérieur d'une surface cylindrique enveloppante prolongée des tubes d'enveloppe (26, 27) et ne dépassent pas radialement ceux-ci.

11. Instrument à électrode (22) selon l'une quelconque des revendications précédentes, dans lequel les tubes d'enveloppe d'électrode (26, 27) sont reliés par l'intermédiaire d'au moins un élément de guidage (25), notamment deux éléments de guidage (25) dans la section distale (33), de préférence avec un premier élément de guidage (25) dans la section distale (33) et un deuxième élément de guidage (25) dans la section proximale (32).

12. Résectoscope (10) avec un instrument à électrode (22) selon l'une quelconque des revendications 1 à 11 précédentes.
